# EUROPÄISCHE PATENTANMELDUNG

(11) **EP 0 622 098 A1**
(43) Veröffentlichungstag der Anmeldung: **02.11.1994**
(21) Anmeldenummer: 94102353.3
(22) Anmeldetag: 17.02.1994
(51) Int. Cl.: A63C 9/00, F16B 41/00

(54) **Haltekörper, insbesondere für Skibindungen**

(30) Priorität: 29.04.1993 AT 838/93
(71) Anmelder: HTM Sport- und Freizeitgeräte Aktiengesellschaft, A-2320 Schwechat (AT)
(72) Erfinder: Zotter, Johann, A-1070 Wien (AT); Leichtfried, Friedrich, Ing., A-2514 Traiskirchen (AT); Hösl, Erwin, A-2405 Hundsheim 105a (AT)
(74) Vertreter: Szász, Tibor, Dipl.-Ing.

(57) **Zusammenfassung**

Die Erfindung betrifft einen Haltekörper für Skibindungen, mit Bohrungen für Befestigungsschrauben, wobei die Wand der Bohrung zum Festhalten der nicht montierten Befestigungsschrauben Ansätze aufweist, welche entlang des Umfanges der Bohrung versetzt angeordnet sind, und welche mit ihren Innenseiten bis zum Kerndurchmesser der einzusetzenden Befestigungsschraube reichen.

Um bei diesem Haltekörper eine einfache Herstellung für die Ansätze und eine leichte Betätigung für die Befestigungsschrauben zu erreichen, sieht die Erfindung vor, daß die Ansätze (6-8) in der Bohrung (4) in derselben Höhe der Wand und in einer den Seiten (9a-c) eines seitengleichen Dreiecks (9) entsprechenden Anordnung vorgesehen sind, wobei die Seiten (9a-c) des Dreiecks zu einem Kreis (K₁), dessen Durchmesser dem Kerndurchmesser (d) der Befestigungsschrauben (5) entspricht, tangential verläuft, und daß die Innenseiten (6a-8a) der einzelnen Ansätze (6-8) in der Draufsicht und in Richtung der Längsmittelachse der Bohrung (4) betrachtet, jeweils entlang eines Bogens verlaufen, welcher durch den von dem Kreis (K₁) und von der dem Bogen zugehörigen Seite (9a-c) des Dreiecks (9)bestimmten Tangentialpunkt geht.

## Beschreibung

Die Erfindung betrifft einen Haltekörper, insbesondere für Skibindungen, mit mindestens einer Bohrung für eine Befestigungsschraube, wobei die Wand der Bohrung zum Festhalten der eingesetzten Befestigungsschraube in deren nicht montierten Zustand Ansätze aufweist, welche entlang des Umfanges der Bohrung relativ zueinander versetzt angeordnet sind.

Ein Haltekörper der eingangs genannten Art ist beispielsweise als eine Grundplatte einer am Markt befindlichen Skibindung bekannt. Bei dieser bekannten Lösung sind in den einzelnen Bohrungen, welche zum Festhalten eingesetzter Befestigungsschrauben dienen, drei Ansätze vorgesehen, welche entlang eines Gewindesganges ausgestaltet sind und welche somit auch in Höhenrichtung relativ zueinander versetzt angeordnet sind. Dabei verläuft die Oberfläche jedes Ansatzes in der Richtung des Gewindeganges und auch der jeweiligen Steigung dieses Gewindeganges entsprechend, um ein einwandfreies Festhalten der einzelnen Befestigungsschrauben zu gewährleisten. Der Nachteil solcherart ausgestalteter Ansätze besteht darin, daß eine Vorrichtung mit Spezialwerkzeugen, welche die Herstellung geeigneter Gewindeabschnitte gewährleisten, verwendet werden muß. Für einen sicheren Halt der Befestigungsschrauben ist bei dieser Haltevorrichtung zwangsläufig die Verwendung dreier Ansätze erforderlich, welche ihrerseits wieder die Verwendung dreier Werkzeuge benötigen, wobei diese noch erodiert werden müssen, um die erforderliche, der Steigung des Gewindes entsprechende Oberfläche der einzelnen Ansätze herstellen zu können. Weiters können die so ausgebildeten Ansätze leicht beschädigt werden, wodurch der sichere Halt und somit die vertikale Lage der einzelnen Befestigungsschrauben gefährdet wird. Dies hat zur Folge, daß anläßlich der Montage die Schrauben in einer von der Vertikalen abweichenden Lage zur Oberseite des Skis stehen, wodurch das gesetze Ziel nicht mehr erreicht wird. Es soll nämlich die Befestigung der Grundplatte einer Skibindung ohne weitere Manipulationen lediglich durch Eindrehen der Befestigungsschrauben in die Halterung, vor allem in den Ski, erfolgen können.

Aus der AT-PS 372 863 ist weiters auch die Maßnahme bekannt, daß zwei Ansätze vorgesehen sind, und daß jeder Ansatz, in der Draufsicht betrachtet, durch zwei, unterschiedliche Radien aufweisende Kreise, von welchen Radien der größere dem Radius der Bohrung entspricht, begrenzt ist, wobei die Kreise einander tangential berühren und die beiden Berührungspunkte der Kreise der beiden Ansätze relativ zueinander umd 180° versetzt sind.

Bei dieser Ausfertigung reicht die Verwendung eines Paares von Ansätzen aus, um die Befestigungsschrauben in den einzelnen Bohrungen in der gewünschten Lage festhalten zu können, wobei die einzelnen Ansätze jedes Paares relativ zueinander entlang des Umfanges der einzelnen Bohrungen um 180° versetzt sind. Dabei wurde eine besonders vorteilhafte Ausführungsform der Erfindung darin gesehen, daß die einzelnen Ansätze in Höhenrichtung im Ausmaß eines (vollen) Gewindeganges der zu Verwendung gelangenden Befestigungs-schraube versetzt sind. Dadurch ist ein besonderer Halt der Befestigungsschrauben in den einzelnen Bohrungen, ohne daß eine unerwünschte Deformation der Ansätze stattfinden würde, gewährleistet.

Die zuletzt genannte Ausgestaltung hat sich in der Praxis vielfach bewährt, ist jedoch mit dem Nachteil der Herstellung der relativ zueinander sowohl entlang des Umfanges der Bohrung als auch in Höhenrichtung versetzten Anordnung der beiden Ansätze behaftet.

Die Erfindung hat sich nun die Aufgabe gestellt, einen Haltekörper der eingangs genannten Art mit derartigen Bohrungen so zu versehen, daß die Ansätze mit ihren Oberseiten in einer gemeinsamen Ebene verlaufend ausgebildet bzw. angeordnet werden können. Dabei soll das Bestimmungsmaterial "Haltekörper" in dieser Anmeldung sowohl einen Trag- oder Grundkörper als auch eine gesonderte Grund- oder Unterlagsplatte einer Skibindung sowie eine Führungsschiene oder ein Podest für eine Skibindung umfassen.

Gelöst wird die gestellte Aufgabe erfindungsgemäß durch die kennzeichnenden Merkmale des Anspruches 1.

Auf diese Weise wird das für das Einschrauben der Befestigungsschraube erforderliche Drehmoment reduziert, weil das tangentiale Anliegen des Schaftes (Kerndurchmessers) der Befestigungsschraube an den einzelnen Ansätzen praktisch punktförmig ist. Des weiteren wird eine einfache Herstellung der Ansätze ermöglicht, da diese nunmehr mit einem einzigen (Folge-)Werkzeug gefertigt werden können.

Die bogenförmige Ausgestaltung der Innenseiten der Ansätze ist besonders dann vorteilhaft, wenn nach den Merkmalen des Anspruches 2 diese jeweils entlang eines Kreisbogens verlaufen und konvex ausgestaltet sind. Diese Ausgestaltung ermöglicht nach Anspruch 3 eine leicht definierbare und genaue Herstellung bei einem Dreieck, Fünfeck oder einem Vieleck, welches durch ein ganzzahliges Merfache des Dreiecks oder Fünfecks entsteht. In den Merkmalen des Anspruches 4 ist eine solche Herstellung bei einem Viereck und bei dessen ganzzahligen Vielfachen gekennzeichnet.

Eine andere vorteilhafte Ausgestaltung ergibt sich durch die Merkmale des Anspruches 5, nach welchen die Anpassung der Ansätze an das Gewinde der jeweils einzusetzenden Befestigungsschraube durch die konkave Ausgestaltung der Innenseiten der Ansätze verwirklicht wird.

Durch die erfindungsgemäße Ausgestaltung der Übergänge der Ansätze nach Anspruch 6 sowie der Abrundungen der einzelner Übergänge nebeneinander liegender Seiten dieser Ansätze und der Übergänge einzelner Seiten mit benachbarten Bögen nach Anspruch 7 wirkt dem Entstehen von Spannungen und Rißbildungen zwischen den Ansätzen und dem Haltekörper entgegen.

Im Anspruch 8 ist dabei eine besonders günstige, praxisbezogene Maßnahme unter Schutz gestellt.

Durch die Merkmale des Anspruches 9 kann beim Einsetzen jeder Befestigungsschraube der sich im jeweiligen Gewinde befindliche Ansatz an seinem freien Endabschnitt aus der gemeinsamen Ebene geringfügig deformiert werden, wodurch sich für die Befestigungsschraube ein noch besserer Halt ergibt.

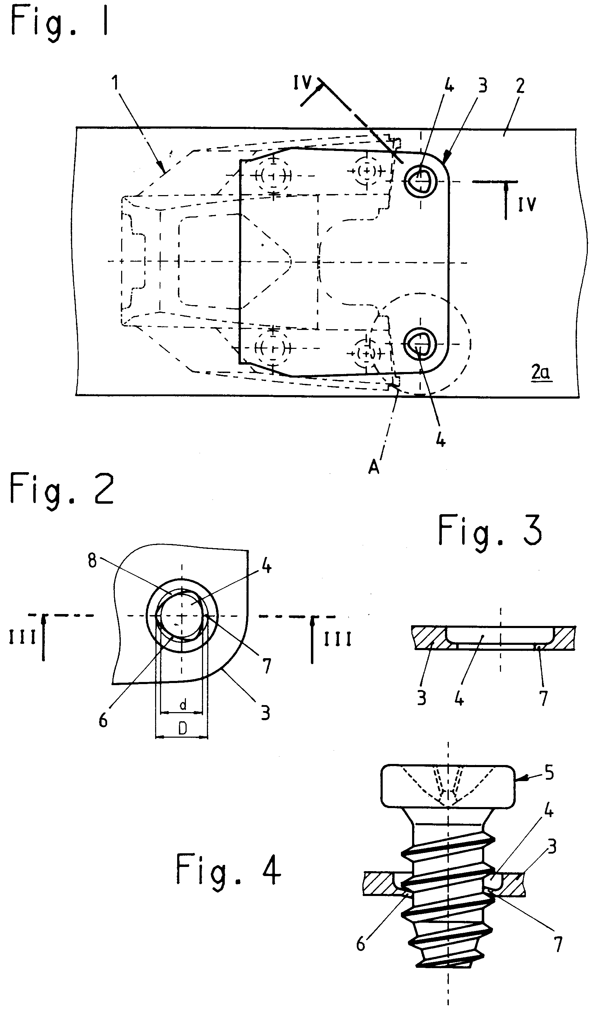

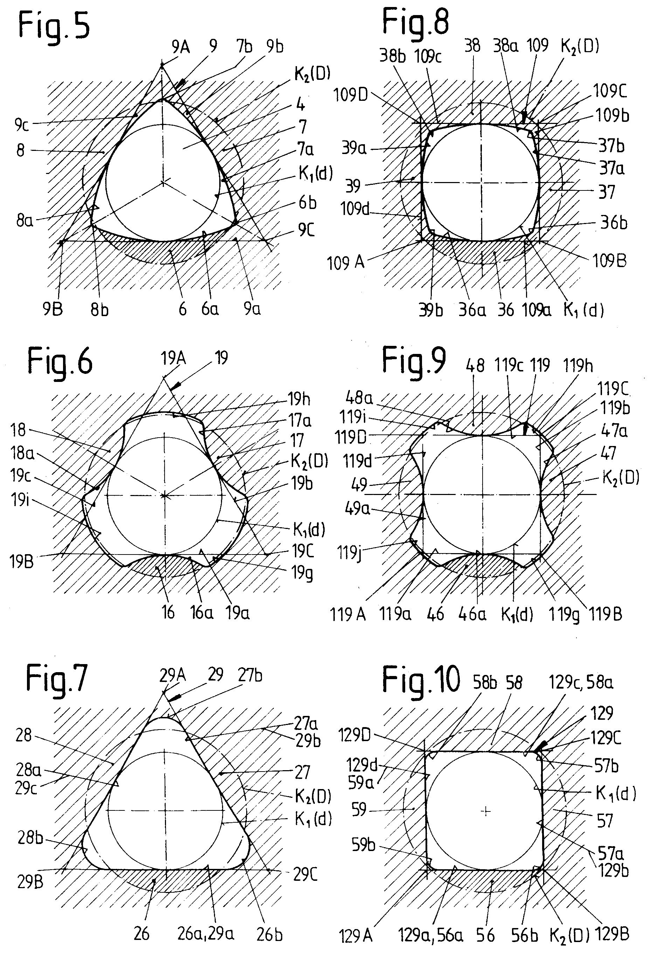

Weitere Merkmale, Einzelheiten und vorzugsweise Ausgestaltungen der Erfindungen ergeben sich an Hand der Zeichnung, in der mehrere Ausführungsbeispiele dargestellt sind. Hiebei zeigen: Die Figuren 1 bis 4 eine erste Ausführungsform der Erfindung, wobei die Fig.1 einen erfindungsgemäßen Haltekörper eines Backenkörpers einer Skbindung in Draufsicht, die Fig.2 das Detail "A" aus Fig.1 in größerem Maßstab, Fig.3 einen Schnitt entlang der Linie III-III in Fig.2 und Fig.4 einen Schnitt entlang der Linie IV-IV in Fig.1 veranschaulicht. Die Figur 5 zeigt die geometrische Ausgestaltung des ersten Ausführungsbeispiels, ähnlich der Fig.2, jedoch in einem noch größerem Maßstab und die Figuren 10 bis 14 weitere Ausführungsbeispiele für die geometrische Ausgestaltung der Erfindung, ähnlich der Fig.5.

Ein in seiner Gesamtheit mit 1 bezeichneter, jedoch nur Strichpunktiert angedeuteter Backenkörper einer Skibindung ist mittels eines als Grundkörper 3 bezeichneten Haltekörpers auf der Oberseite 2a eines Ski 2 befestigt. Zur Befestigung des Grundkörpers 3 auf der Oberseite 2a des Ski 2 dienen Befestigungsschrauben 5, von welchen eine in der Fig.4 in eine der Bohrungen 4 des Grundkörpers 3 eingesetzt dargestellt ist. Wie man es der Fig.1 entnehmen kann, sind zur Befestigung des Grundkörpers 3 auf der Oberseite 2a des Ski 2 zwei Bohrungen 4 vorgesehen. Dabei sind drei Ansätze 6, 7, 8 vorgesehen, deren Ausgestaltung besser den Figuren 2 und 3 entnommen werden kann.

Die geometrische Ausgestaltung der ersten Ausführungsform nach den Figuren 1 bis 4 ist in Details in der noch zu beschreibenden Fig.5, die etwa in einem Maßstab 8:1 erstellt worden ist, dargestellt. Aus dieser Figur können die die einzelnen Teilbereiche bezeichnenden Bezugszeichen besser identifiziert werden.

In jeder Bohrung 4 verlaufen alle Ansätze 6,7,8 - im fertigen Grundkörper 3 - mit ihren Unter- und Oberseiten jeweils in einer Ebene und sind - im Querschnitt betrachtet - fluchtend zueinander angeordnet (vgl. Fig.3). Dabei sind die Ansätze 6,7,8 so ausgebildet, daß ihre Innenseiten 6a,7a,8a, in der Draufsicht und in Richtung zur Längsmittelachse der Bohrung 4 betrachtet, durch die Seiten 9a,9b,9c eines seitengleichen Dreiecks 9 bestimmt werden, wie dies an Hand der Fig.5 noch näher erläutert werden wird. Um das Entstehen von lokalen Überbeanspruchungen beim Einsetzen und Festziehen der Befestigungsschraube 5 zu verhindern, sind die aneinander angrenzenden Innenseiten 6a,7a,8a der Ansätze 6,7,8 an ihren Übergängen jeweils mit Abrundungen 6b,7b,8b gestaltet. Des weiteren ist, wie ein Vergleich auch mit Fig.2 erkennen läßt, die Ausgestaltung derart, daß der Kerndurchmesser d der einzusetzenden Befestigungsschraube 5 dem Durchmesser eines in das Dreieck 9 eingeschriebenen Kreises K₁ entspricht. In der Fig.2 ist der Durchmesser der Bohrung 4 mit D bezeichnet. Dieser entspricht dem Nenndurchmesser der einzusetzenden Befestigungsschraube 5.

Wie man es der Fig.3 entnehmen kann, ist die Dicke bzw. Stärke der einzelnen Ansätze gering; sie beträgt in der Praxis 0,3 - 0,6 mm. Vorzugsweise werden die Ansätze in einer Stärke von 0,4 mm gefestigt. Dadurch wird gewährleistet, daß beim Einsetzen der Befestigungsschraube 4 die freien Endabschnitte der einzelnen Ansätze 6,7,8 leicht deformiert werden (vgl. Fig.4), wodurch sich für die Befestigungsschraube 4 für den Transport und für das Eindrehen eine definierte, gute Halterung ergibt.

An Hand der Fig.5 wird nun die geometrische Ausgestaltung des ersten Ausführungsbeispiels näher erläutert. Ausgehend aus dem Kerndurchmesser d der einzusetzenden Befestigungsschraube 5 wird das seitengleiche Dreieck 9 in bekannter Weise so konstruiert, daß es mit den Mittelpunkten seiner Seiten 9a,9b,9c den Kreis K₁ tangential berührt. Nun werden von den Eckpunkten 9A,9B,9C des Dreiecks 9 ausgehend an die jeweils gegenüberliegenden Berührungspunkte Kreisbögen gezogen, welche dem Verlauf der Innenseiten 6a,7a,8a der einzelnen Ansätze 6,7,8 entsprechen. Dabei wurde - der besseren Übersicht halber - in der Fig.5 nur der eine Ansatz 6 mit enger Schraffur hervorgehoben. Es ist weiters erkenn-bar, daß die Innenseiten 6a,7a,8a der Ansätze 6,7,8 ihren Schnittpunkt geringfügig außerhalb eines zweiten Kreises K₂ haben, dessen Durchmesser dem Nenndurchmesser D der einzu-setzenden Befestigungsschraube 5 entspricht (s.Fig.2). Es ist aber ohne weiteres denkbar, den Zusammenlauf der einzelnen Bögen bzw. Innenseiten der Ansätze 6,7,8 genau dem Nenndurchmesser der einzusetzenden Befestigungsschraube 5 anzupassen.

Wichtiger und erfindungswesentlich ist, daß durch diese Ausgestaltung jede einzusetzende Befestigungsschraube 5 an dem ihr zugehörigen Ansatz - in der Draufsicht und in Richtung zur Längsmittelachse der Bohrung 4 betrachtet - etwa punktförmig anliegt, wodurch beim Einschrauben der Befestigungsschrauben nur ein geringes Drehmoment zu überwunden ist, demgegenüber im Haltekörper 4 für diese Befestigungsschraube 5 ein definierter und sicherer Halt gewährleistet wird.

In den Ausführungsbeispielen nach den Figuren 6 und 7 wird zur Bestimmung der Ansätze wiederum ein seitengleiches Dreieck 19 bzw. 29 verwendet. Dabei sind die Anordnungen zwischen dem Dreieck 19 bzw. 29 und dem Kreis K₁ gleich mit der Ausgestaltung des ersten Ausführungsbeispiels. Ein Unter-schied besteht in der Ausführungsform der Ansätze, indem die Innenseiten 16a,17a, 18a der Ansätze 16,17,18 entlang von Kreisbögen verlaufen, deren Mittelpunkt jeweils an einer Normalen liegt, welche durch den Berührungspunkt der Mitte der zugehörigen Seite 19a,19b,19c des Dreiecks 19 und der Längsmittelachse der Bohrung 4 verläuft. In der Ausführungsform nach der Fig.7 sind die Innenseiten 26a,27a,28a der Ansätze 26,27,28 durch die Seiten 29a,29b,29c des seitengleichen Dreiecks 29 selbst gebildet.

Aus herstellungstechnischen Gründen ist der Übergang der nebeneinanderliegenden Seiten in den Ausführungsbeispielen nach den Figuren 5 und 7 mit Abrundungen 6b,7b,8b bzw. 26b, 27b,28b gebildet. In der Ausführungsform nach der Fig.6 sind die freien Endabschitte der nebeneinanderliegenden Ansätze 16,17,18 mit einzelnen Bögen 19g,19h,19i miteinander verbunden, wobei die einzelnen Übergänge auch in diesem Fall abgerundet sind.

Auch in den Ausführungsformen nach den Figuren 6 und 7 ist die Anlage der einzusetzenden Befestigungsschraube 5 an den jeweiligen Ansätzen 16,17,18 bzw. 26,27,28, ähnlich wie im ersten Ausführungsbeispiel, praktisch punktförmig. Die bereits zum ersten Ausführungsbeispiel angeführten Vorteile sind daher bei diesen Ausgestaltungen ebenfalls vorhanden.

Die Figuren 8 bis 10 zeigen Ausgestaltungen, die mit Hilfe von regulären Vierecken (Quadraten) 109,119,129 erstellt werden, wobei hinsichtlich der Form der Ansätze 36, 37,38,39 bzw. 46,47,48,49 bzw. 56, 57, 58, 59 diese der Reihe nach jenen nach den Figuren 5 bis 7 ähneln. Hinsichtlich der geometrischen Ausgestaltung besteht insoferne ein Unterschied als im Ausführungsbeispiel nach der Fig.8 der Mittelpunkt der Innenseite 36a,37a,38a,39a, der einzelnen Ansätze 36,37,38, 39 am jeweils gegenüberliegenden Berührungspunkt vom Kreis K₁ und der zugehörigen Seite 109a,109b,109c,109d des Vierecks (Quadrats) 109 liegt.

Auch bei diesen Ausführungsbeispielen ist jeweils eine annähernd punktförmige Anlage des Kerndurchmessers d der einzusetzenden Befestigungsschraube 5 an den einzelnen Ansätzen gewährleistet, allerdings mit einem geringfügigen Nachteil, als in diesem Fall, da vier Ansätze vorhanden sind, das zu überwindende Drehmoment etwas erhöht wird. Dieser geringfügige Nachteil wird jedoch durch die vierfache Abstützung kompensiert.

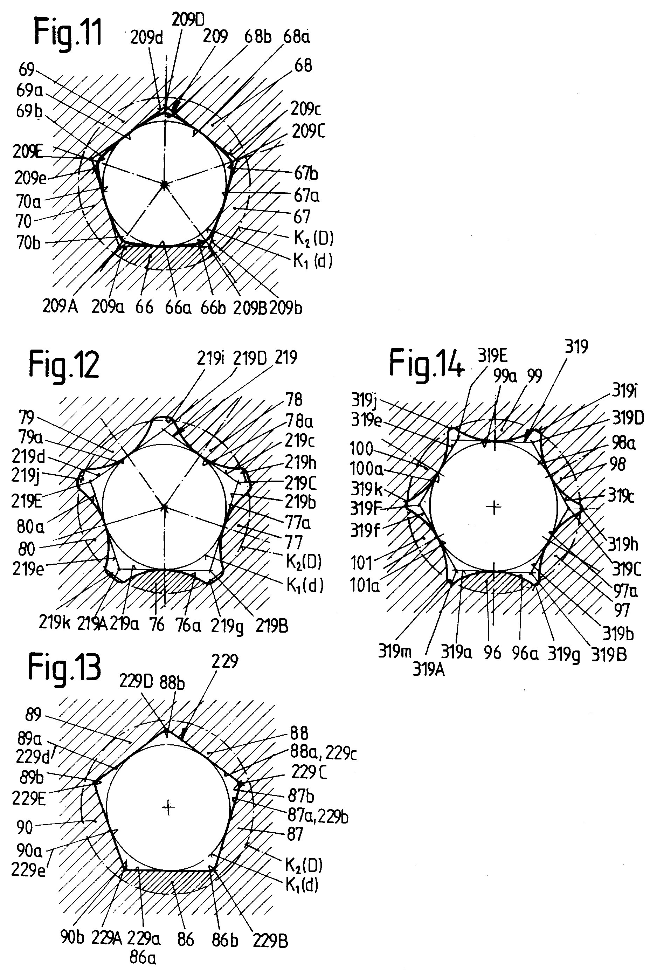

Um die Verwendung des erfindungsgemäßen Gedankens auch für weitere reguläre Vielecke zu veranschaulichen, wurden in den Figuren 11 bis 13 Ausgestaltungen in Verbindung mit einem regulären Fünfeck 209,219,229 und in der Fig.14 mit einem regulären Sechseck 319 dargestellt. Dabei entspricht die Ausgestaltung der Ansätze 66-70; 76-80; 86-90 bzw. 96-101 in den Figuren 11 bis 13 der Reihe nach jener nach den Figuren 5 bis 7 und in der Fig.14 jener nach der Fig.6. Auf eine detaillierte Beschreibung wurde verzichtet, da es sich für den Fachmann an Hand der Bestimmungsmerkmale der beiliegenden Stückliste um eine leicht nachvollziehbare Lehre handelt.

Auch die Ausgestaltung des vielfachen eines Dreiecks oder regulären Vierecks oder Fünfecks bedarf es nicht, weil es sich sich dabei um für den Fachmann leicht nachvollziehbare elementar-geometrische Kenntnisse handelt. Die Darstellung eines Sechsecks erfolgte in diesem Zusammenhang ausschließlich aus Gründen einer ersten Anleitung.

Wenn auch ganzzahlige mehrfache eines Dreiecks oder regulären Vielecks durchaus im Rahmen der Erfindung liegen sollen, so ist es zu erwähnen, daß mit steigender Anzahl der Anlagepunkte des Kerndurchmessers der einzusetzenden Befestigungsschraube an den Ansätzen - d.h. mit steigender Anzahl der Ansätze - der Effekt des geringen Drehmomentes an seiner Wirkung verliert. Trotzdem kann es in gewissen Fällen vorteilhaft sein, die Anzahl der Abstützungen zu erhöhen. Durch die Erfindung sollen daher auch derartige Ausgestaltungen geschützt werden.

Es sei in diesem Zusammenhang wiederholt betont werden, daß die geringe Anlageflächen zwischen der Befestigungsschraube und den sie haltenden Ansätzen die wichtigste Maßnahme der vorliegenden Erfindung ist.

Die beschriebene Halterung für Befestigungsschrauben ist nicht nur hinsichtlich der Herstellung einfach, sie zeigt auch überraschender Weise eine gut definierte Lage für die Befestigungsschrauben und verlängert auch die Lebensdauer der zur Herstellung benötigten Werkzeuge. Es wurde bereits eingangs erwähnt, daß durch die erfindungsgemäße Maßnahme die Herstellung der Ansätze in einem einzigen Arbeitsgang erfolgen kann, durch die geringe Stärke der Ansätze wird zuzüglich die für das Einsetzen der Befestigungsschrauben erforderliche Zeit (der Arbeitsaufwand) verringert.

Die Erfindung ist nicht auf das beschriebene und in der Zeichnung gezeigte Ausführungsbeispiel beschränkt. Es sind weitere Abwandlungen denkbar, ohne den Schutzumfang der Erfindung zu verlassen.

## Patentansprüche

1. Haltekörper, insbesondere für Skibindungen, mit mindestens einer Bohrung für eine Befestigungsschraube (5), wobei die Wand der Bohrung (4) zum Festhalten der Befestigungsschraube (5) in deren nicht montierten Zustand Ansätze (6-8, 16-18, 26-28; 36-39, 46-49, 56-59; 66-70, 76-80; 86-90; 96-101) aufweist, welche entlang des Umfanges der Bohrung (4) relativ zueinander in gleichen Abständen versetzt angeordnet sind, und welche mit ihren Innenseiten in Richtung zur Längsmittelachse der Bohrung (4) hin weisend bis zum Kerndurchmesser der einzusetzenden Befestigungsschraube (5) reichen, dadurch gekennzeichnet, daß die Ansätze (6-8, 16-18, 26-28; 36-39, 46-49, 56-59; 66-70, 76-80; 86-90; 96-101) in der Bohrung (4) in ein und derselben Höhe der Wand und in einer den Seiten (9a-c, 19a-c, 29a-c; 109a-d, 119a-d, 129a-d; 209a-e, 219a-e; 319a-f) eines seitengleichen Dreiecks (9,19, 29) oder regulären Vielecks (109,119,129; 209,219,229; 319) entsprechenden Anordnung vorgesehen sind, wobei das Dreieck oder Vieleck mit seinen einzelnen Seiten (9a-c, 19a-c, 29a-c; 109a-d, 119a-d, 129a-d; 209a-e, 219a-e, 229a-e; 319a-f) zu einem ideellen Kreis (K₁), dessen Durchmesser dem Kerndurchmesser (d) der in die Bohrung einzusetzenden Befestigungsschraube (5) entspricht, tangential verläuft, und daß vorzugsweise die Innenseiten (6a-8a, 16a-18a, 26a-28a; 36a-39a, 46a-49a, 56a-59a; 209a-209e, 219a-219e, 229a-229e; 319a-319f) der einzelnen Ansätze (6-8, 16-18, 26-28; 36-39, 46-49, 56-59; 66-70, 76-80; 86-90; 96-101)) mit den Seiten (9a-c, 19a-c, 29a-c; 109a-d, 119a-d, 129a-d, 209a-e, 219a-e, 229a-e; 319a-f) des Dreiecks (9,19,29) oder Vielecks (109,119,129; 209,219,229; 319)), in der Draufsicht und in Richtung der Längsmittelachse der Bohrung (4) betrachtet, jeweils entlang eines Bogens verlaufen, welcher durch den von dem Kreis (K₁) und von der dem Bogen zugehörigen Seite (9a-c, 19a-c, 29a-c; 109a-d, 119a-d, 129a-d; 209a-e, 219a-e, 229a-e; 319a-f) des Dreiecks (9,19,29) oder Vielecks (109,119,129; 209,219,229,; 319) bestimmten Tangentialpunkt geht (Fig.7,10,13; 5,6, 8,9,11,12,14).

2. Haltekörper nach Anspruch 1, dadurch gekennzeichnet, daß die bogenförmigen Innenseiten (6a-8a, 16a-18a, 26a-28a; 36a-39a, 46a-49a, 56a-59a; 209a-209e, 219a-219e, 229a-229e; 319-319f) der einzelnen Ansätze (6-8, 16-18, 26-28; 36-39, 46-49, 56-59; 66-70, 76-80; 86-90; 86-101)) jeweils entlang eines Kreisbogens verlaufen, und daß jeder Kreisbogen - von der Längsmittelachse der Bohrung aus betrachtet - einen konvexen Verlauf aufweist (Fig.5,8,11).

3. Haltekörper nach Anspruch 1 oder 2, dadurch gekennzeichnet, daß bei einer Anordnung der Ansätze (6-8; 66-70) gemäß einem seitengleichen Dreieck (9), regulären Fünfeck (209) oder regulären Vieleck, welches letztere durch ein ganzzahliges Mehrfache des Dreiecks oder Fünfecks gebildet ist, der Mittelpunkt (je)des den Verlauf der Innenseiten (6a-8a; 66a-70a) der einzelnen Ansätze (6-8; 66-70) bestimmenden Kreisbogens am der jeweiligen Seite (9a, 9b, 9c) gegenüberliegenden Eckpunkt (9A, 9B, 9C) dieses Dreiecks (9), Fünfecks (209) oder Vielecks liegt (Fig.5,11).

4. Haltekörper nach Anspruch 1 oder 2, dadurch gekennzeichnet, daß bei einer Anordnung der Ansätze (36-39) gemäß einem regulären Viereck (109) oder regulären Vieleck, welches letztere durch ein ganzzahliges Mehrfache dieses Vierecks gebildet ist, der Mittelpunkt (je)des den Verlauf der Innenseiten (36a-39a) der einzelnen Ansätze (36-39) bestimmenden Kreisbogens am diesem Kreisbogen gegenüber-liegenden Berührungspunkt dieses Vierecks (109) oder Vielecks mit dem Kreis (K₁) liegt, dessen Durchmesser dem Kerndurchmesser (d) der einzusetzenden Befestigungsschraube (5) entspricht (Fig.8).

5. Haltekörper nach Anspruch 1, dadurch gekennzeichnet, daß der Mittelpunkt jedes Kreisbogens an einer Normalen liegt, die sowohl durch den Mittelpunkt jenes Kreises (K₁) geht, dessen Druchmesser dem Kerndurchmesser 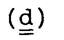 der in die Bohrung (4) einzusetzenden Befestigungsschraube (5) entspricht, als auch durch die Mitte der dem jeweiligen Kreisbogen zugehörigen Seite (19a-c; 119a-d; 219a-e; 319a-f) des Dreiecks (19) oder Vielecks (119,219,319) geht, und daß jeder Kreisbogen - von der Längsmittelachse der Bohrung aus betrachtet - einen konkaven Verlauf aufweist (Fig.6,9,12 und 14).

6. Haltekörper nach einem der Ansprüche 1 bis 5, dadurch gekennzeichnet, daß die jeweils nebeneinander liegenden Kreisbögen entweder unmittelbar ineinander übergehen, oder jeweils mit einem weiteren Bogen (19g-i; 119g-j; 219g-k; 319g-m) miteinander verbunden sind, welche letztere an einem weiteren Kreis anliegen, dessen Durchmesser gleich groß wie oder geringfügig größer als der Nenndurchmesser (D) der einzusetzenden Befestigungsschraube (5) ist (Fig.6,7,9,12 und 14).

7. Haltekörper nach einem der Ansprüche 1 bis 6, dadurch gekennzeichnet, daß der Übergang nebeneinander liegender Innenseiten zweier benachbarter Ansätze oder der Übergang zwischen der Innenseite eines Ansatzes und dem anschließenden Kreisbogen jeweils mit einer Abrundung gebildet ist.

8. Haltekörper nach Anspruch 7, dadurch gekennzeichnet, daß jede Abrundung (6b-8b, 26b-28b; 36b-39b, 56b-59b; 66b-70b; 86b-90b) der einzelnen nebeneinander liegenden Ansätze (6-8, 26-28; 36-39, 56-59; 66-70, 86-90)) mit einem Radius (R) von 0,5 bis 1,5 mm, vorzugsweise mit 1mm, gebildet ist.

9. Haltekörper nach einem der Ansprüche 1 bis 8, dadurch gekennzeichnet, daß die Dicke bzw. Stärke der einzelnen Ansätze (6-8, 16-18, 26-28; 36-39, 46-49, 56-59; 66-70, 76-80; 86-90; 96-101) 0,3 - 0,6 mm, vorzugsweise 0,4 mm, beträgt.
